(19) Europäisches Patentamt
European Patent Office
Office européen des brevets

(11) Veröffentlichungsnummer: **0 479 178 A2**

(12) **EUROPÄISCHE PATENTANMELDUNG**

(21) Anmeldenummer: **91116633.8**

(22) Anmeldetag: **30.09.91**

(51) Int. Cl.[5]: **C07D 213/79**, A61K 31/44, C07D 213/803

(30) Priorität: **01.10.90 DE 4030999**

(43) Veröffentlichungstag der Anmeldung: **08.04.92 Patentblatt 92/15**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Baader, Ekkehard, Dr.**
**Amselweg 14**
**W-6240 Königstein/Taunus(DE)**
Erfinder: **Bickel, Martin, Dr.**
**Mittelstedter Weg 3**
**W-6380 Bad Homburg(DE)**
Erfinder: **Günzler-Pukall, Volkmar, Dr.**
**Gross-Seelheimer Strasse 13**
**W-3550 Marburg(DE)**

(54) **4- oder 5-substituierte Pyridin-2-carbonsäuren, Verfahren zu deren Herstellung sowie deren Verwendung als Arzneimittel.**

(57) Die Erfindung betrifft in 4- oder 5-Stellung mit Carboxylgruppen substituierte Pyridin-2-carbonsäuren und ihre Verwendung als Arzneimittel.

Insbesondere wird die Wirkung der Verbindungen zur Inhibierung der Prolin- und Lysinhydroxylase und zur Behandlung von Störungen der Biosynthese von Kollagen und kollagenähnlichen Stoffen beschrieben.

EP 0 479 178 A2

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren wie $\alpha,\alpha'$-Dipyridyl zu einer Hemmung der C1q-Biosynthese von Makrophagen führt (W. Müller et al., FEBS Lett. 90 (1978), 218; Immunobiology 155 (1978), 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß das Enzym Prolinhydroxylase durch Pyridin-2,4- und -2,5-dicarbonsäuren effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 248 (1987) 625-633).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und -2,5-dicarbonsäuren, die die Kollagenbiosynthese im Tiermodell wirksam hemmen. So wird in der DE-A 37 03 959 unter anderem die Synthese von N,N'-Bis(2-methoxyethyl)-pyridin-2,4-dicarbonsäurediamid und N,N'-Bis(3-isopropoxypropyl)-pyridin-2,4-dicarbonsäurediamid beschrieben.

In den deutschen Patentanmeldungen P 38 26 471.4 und P 38 28 140.6 wird ein verbessertes Verfahren zur Herstellung von N,N'-Bis(2-methoxyethyl)-pyridin-2,4-dicarbonsäurediamidvorgeschlagen.

Die deutsche Patentanmeldung P 39 24 093.2 schlägt neue N,N'-Bis(alkoxyalkyl)-pyridin-2,4-dicarbonsäurediamide vor.

Die deutsche Patentanmeldung P 40 01 002.3 beschreibt die Verwendung von Pyridin-2,4- und 2,5-dicarbonsäuredi(nitroxyalkyl)amide zur Herstellung von Prolin- und Lysinhydroxylase hemmenden Arzneimitteln.

Sowohl Pyridin-2,4- und -2,5-dicarbonsäurediamid (Hirakata et al., J. pharm. Soc. Japan 77 (1957) 219 und Häring et al., Helv. 37 (1954) 147, 153) als auch Pyridin-2,4 und -2,5-dicarbonsäuredihydrazid (Itai et al., Bl. nation. hyg. Labor. Tokyo, 74 (1956) 115, 117 und Shinohara et al., Chem. High Polymers Japan, 15 (1958) 839) sind bereits als Tuberkulosemittel bekannt.

In der JP 53/28175 (78/28175) werden N,N'-bis(2-nitrooxyethyl)pyridin-2,4- und -2,5-dicarbonsäurediamide als Substanzen mit vasodilatorischer Wirkung beschrieben.

Die deutsche Patentanmeldung P 40 20 570.3 beschreibt die Verwendung von 2,4- und 2,5-substituierten Pyridin-N-oxiden zur Herstellung von Prolin- und Lysinhydroxylase hemmenden Arzneimitteln.

Überraschend wurde nun gefunden, daß 4- oder 5-substituierte Pyridin-2-carbonsäuren der unten angegebenen allgemeinen Formel I sowie deren phyiologisch verträglichen Salze die Lysin- und Prolinhydroxylase im Tiermodell wirksam inhibieren.

Erfindungsgemäß beansprucht werden daher Verbindungen gemäß Formel I

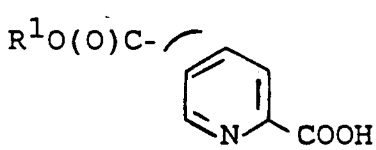

(I)

worin

$R^1$

$C_1$-$C_{12}$-Alkyl, $C_2$-$C_{12}$-Alkenyl, $C_2$-$C_{12}$-Alkynyl, nichtbenzoanneliertes oder benzoanneliertes $C_5$-$C_7$-Cycloalkyl, Aryl oder Heteroaryl bedeutet, wobei dieses für $R^1$ genannten Rest unsubstituiert ist oder substituiert ist

mit einem oder mehreren gleichen oder unterschiedlichen Resten $R^2$, wobei

$R^2$ Halogen, Hydroxy, Cyano, Nitro, Nitroxy, Amino, Carboxyl, $C_1$-$C_4$-Alkoxy, $C_1$-$C_4$-Alkoxycarbonyl, $C_1$-$C_4$-Alkyl- oder dialkylamino, Indolyl oder Phenyl bedeutet, wobei der Indolyl- und Phenylrest unsubstituiert ist oder 1-, 2- oder 3-fach substituiert ist mit Halogen, Nitro, $C_1$-$C_4$-Alkyl oder $C_1$-$C_4$-Alkoxy, wobei bei Mehrfachsubstitition die Reste gleich oder verschieden sind sowie die physiologisch verträglichen Salze, wobei die 4-Methyl- und -Benzylester der 2,4- und die 5-Methyl- und -Benzylester der 2,5-Pyridindicarbonsäure ausgenommen sind.

Insbesondere bevorzugt sind Verbindungen der allgemeinen Formel I, worin

$R^1$ $C_1$-$C_6$-Alkyl, $C_2$-$C_6$-Alkenyl, $C_2$-$C_6$-Alkynyl, $C_5$-$C_7$-Cycloalkyl, Aryl oder Heteroaryl bedeutet, wobei diese für $R^1$ genannten Reste unsubstituiert sind oder substituiert sind mit einem oder zwei gleichen oder unterschiedlichen Resten $R^2$, wobei

$R^2$ Halogen, Hydroxy, Cyano, Amino, Carboxyl, $C_1$-$C_4$-Alkoxy, $C_1$-$C_4$-Alkoxycarbonyl, $C_1$-$C_4$-Alkyl- oder dialkylamino, oder Phenyl bedeutet, wobei der Phenylrest unsubstituiert ist oder 1-fach substituiert ist mit Halogen, $C_1$-$C_2$-Alkyl oder $C_1$-$C_2$-Alkoxy, sowie die physiologisch verträglichen Salzen, wobei die 4-Methyl- und -Benzylester der 2,4- und die 5-Methyl- und -Benzylester der 2,5-Pyridindicarbonsäure ausgenommen sind.

Besonders bevorzugt sind Verbindungen der allgemeinen Formel I, worin

$R^1$ $C_1$-$C_5$-Alkyl, $C_6$-Cycloalkyl, Phenyl oder Pyridyl bedeutet, wobei diese für $R^1$ genannten Reste unsubstituiert sind oder substituiert sind mit einem oder zwei gleichen Resten $R^2$, wobei

$R^2$ Hydroxy, Amino, Carboxyl, $C_1$-$C_4$-Alkoxy, $C_1$-$C_4$-Alkoxycarbonyl, oder Phenyl bedeutet, wobei der Phenylrest unsubstituiert ist oder 1-fach substituiert ist mit Methyl oder Methoxy, sowie die physiologisch verträglichen Salze, wobei die 4-Methyl- und -Benzylester der 2,4- und die 5-Methyl- und -Benzylester der 2,5-Pyridindicarbonsäure ausgenommen sind.

Die genannten 4-Methyl- und -Benzylester der 2,4- und die 5-Methyl- und -Benzylester der 2,5-Pyridindicarbonsäure werden beschrieben von Thums, L: J. Pharm. Biolog. 24 (1-2), 3-21 (1969) und Delarge, J: Pharm. Acta. Helv. 44 (10), 637-43 (1969). Die entsprechenden 4-Benzylester sind aus der P ......... (HOE 89/F 241) und die 5-Benzylester aus der DE-A-37 03 963 bekannt.

Unter Halogen werden Fluor, Chlor, Brom und Jod, unter Aryl Phenyl und Naphthyl und unter Heteroaryl 5- und 6-gliedrige aromatische Ringe mit 1, 2 oder 3 Stickstoff- und/oder Sauerstoff- und/oder Schwefelatomen verstanden, die gegebenenfalls noch benzoanneliert sein können; insbesondere handelt es sich bei den Heteroarylresten um Pyridyl-, Pyridazyl-, Pyrimidyl-, Pyrazyl-, 1,3,5-Triazyl-, Pyrrolyl-, Pyrazolyl-, Imidazolyl-, Triazolyl-, Tetrazolyl-, Thienyl-, Oxazolyl- und Thiazolyl-Reste und gegebenenfalls deren benzoannlierte Verbindungen.

"Mehrfach substituiert" bedeutet im Vorstehenden und Folgenden, daß mindestens 2 höchstens alle in den Alkylresten vorhanden Wasserstoffatome durch die genannten Substituenten ersetzt sind. Bevorzugt handelt es sich dabei um einen Substituenten pro Methyl- bzw. Methylengruppe.

Bei Mehrfachsubstitution können die Substituenten auch voneinander unabhängig verschieden sein.

Weiterhin betrifft die Erfindung die Verwendung von Verbindungen der allgemeinen Formel I zuzüglich die 4-Methyl- und -Benzylester der 2,4- und die 5-Methyl- und -Benzylester der 2,5-Pyridindicarbonsäure sowie deren physiologisch verträglichen Salze zur Herstellung eines Prolin- und Lysinhydroxylase hemmenden Arzneimittels.

Schließlich betrifft die Erfindung die Verbindungen der allgemeinen Formel I zuzüglich die 4-Methyl- und -Benzylester der 2,4- und die 5-Methyl- und -Benzylester der 2,5-Pyridindicarbonsäure zur Verwendung als Arzneimittel.

Insbesondere betrifft die Erfindung die Verbindungen der Formel I zuzüglich die 4-Methyl- und -Benzylester der 2,4- und die 5-Methyl- und -Benzylester der 2,5-Pyridindicarbonsäure zur Anwendung als Fibrosuppressiva und Immunsuppressiva sowie zur Inhibierung der Prolin- und Lysinhydroxylase und zur Beeinflussung des Stoffwechsels von Kollagen und kollagenähnlichen Stoffen bzw. der Biosynthese von C1q.

Alle genannten Alkylreste mit mehr als 2 C-Atomen können sowohl geradkettig als auch verzweigt sein.

Weiterhin betrifft die Erfindung ein Verfahren zur Herstellung von Verbindungen der allgemeinen Formel I, zuzüglich der 4-Methyl- und -Benzylester der 2,4- und die 5-Methyl- und -Benzylester der 2,5-Pyridindicarbonsäure, das dadurch gekennzeichnet ist, daß man eine Verbindung der allgemeinen Formel II

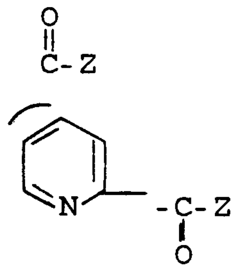

(II),

worin

Z Halogen, insbesondere Chlor oder Brom, Hydroxy oder Alkoxy, insbesondere ($C_1$-$C_6$)-Alkoxy, bedeutet

mit einer Verbindung der allgemeinen Formel III

$HO\text{-}R^1$ (III)

umsetzt,

worin

$R^1$ die in Anspruch 1 angegebene Bedeutung hat, und in einer zweiten Reaktionsstufe den daraus entstandenen Diester der allgemeinen Formel IV

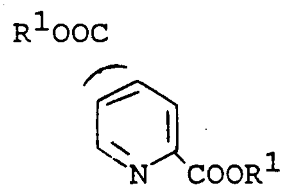

(IV)

in 2-Stellung selektiv verseift.

Die selektive Verseifung eines Esters z.B. in Gegenwart eines Kupfer-Katalysators wird von Delarge, J. in: Pharm. Acta. Helv. 44 (10), 637 (1969) beschrieben.

Das genannte Verfahren ist zur Herstellung der entsprechenden Benzylester aus der DE-A-37 03 963 bekannt.

Die erfindungsgemäßen Verbindungen der Formel I besitzen wertvolle pharmakologische Eigenschaften und zeigen insbesondere Wirksamkeit als Hemmer der Prolin- und Lysinhydroxylase, als Fibrosuppressivum und Immunsuppressivum.

Die Aktivität der Fibrogenase kann durch radioimmunologische Bestimmung des N-terminalen Propeptids des Kollagens Typ-III oder der N- bzw. C-terminalen Quervernetzungsdomäne des Kollagens Typ-IV (7s-Kollagen bzw. Typ-IV-Kollagen-$NC_1$) im Serum bestimmt werden.

Zu diesem Zweck wurden die Hydroxyprolin-, Prokollagen-III-Peptid-, 7s-Kollagen- und Typ-IV-Kollagen-$NC_1$-Konzentration in der Leber von

a) unbehandelten Ratten (Kontrolle)

b) Ratten, denen Tetrachlorkohlenstoff verabreicht wurde ($CCl_4$-Kontrolle)

c) Ratten, denen zunächst $CCl_4$ und anschließend eine erfindungsgemäße Verbindung verabreicht wurde

gemessen (diese Testmethode wird beschrieben von Roullier, C., experimental toxic injury of the liver; in The Liver C. Roullier, Vol. 2, S. 335-476, New York, Academic Press, 1964).

Die Verbindungen der Formel I zuzüglich der 4-Methyl- und -Benzylester der 2,4- und die 5-Methyl- und -Benzylester der 2,5-Pyridindicarbonsäure können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,01 - 25,0 mg/kg/Tag, vorzugsweise 0,01 - 5,0 mg/kg/Tag oder parenteral in Dosen von 0,001 - 5 mg/kg/Tag, vorzugsweise 0,001 - 2,5 mg/kg/Tag,

insbesondere 0,005 - 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimittel, die ein oder mehrere erfindungsgemäße Verbindungen der Formel I und/oder deren physiologisch verträglichen Salze enthalten, zuzüglich der 4-Methyl- und -Benzylester der 2,4- und die 5-Methyl- und -Benzylester der 2,5-Pyridindicarbonsäure.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 % vorteilhafterweise zwischen 10 und 75 % beträgt.

Geeignete Hilfs- bzw. Trägerstoffe für die gewünschte Arzneimittelformulierung sind beispielsweise neben Lösemitteln, Gel bindern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral oder rektal appliziert werden.

Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige, alkoholische oder ölige Suspensionen oder wäßrige oder ölige Lösungen.

Als inerte Trägerstoffe können z. B. Gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung an Hand von Beispielen näher erläutert.

Allgemeine Vorschrift zur Herstellung von erfindungsgemäßen Verbindungen

0,1 mol Kupfernitrat (X 3 $H_2O$) werden in 700 ml Methanol vorgelegt und 0,1 mol eines Pyridin-2,4-oder 2,5-dicarbonsäureesters der allgemeinen Formel IV zugegeben. Es wird 3 Stunden unter Rückfluß erhitzt, abgekühlt, der entstandene Kupferkomplex abgesaugt und mit Diethylether nachgewaschen.
0,1 mol des Kupferkomplexes werden in 400 ml Dioxan vorgelegt. Über einen Zeitraum von 2 Stunden wird bei Raumtemperatur Schwefelwasserstoff eingeleitet. Das ausgefallene Kupfersulfid wird abfiltriert, der Filter mit wenig Dioxan gewaschen. Das Filtrat wird eingeengt, der Rückstand mit Petrolether verrieben und der kristalline Rückstand abfiltriert.

**Beispiel 1**

Pyridin-2-carbonsäure-4-carbonsäurebenzylester

Ausbeute: 77%  Schmelzpunkt: 113-115 ° C

**Beispiel 2**

Pyridin-2-carbonsäure-5-carbonsäurebenzylester

Ausbeute: 75%  Schmelzpunkt: 132 ° C

**Beispiel 3**

Pyridin-2-carbonsäure-4-carbonsäureethylester

Ausbeute: 96%  Schmelzpunkt: 149-153 ° C

**Beispiel 4**

Pyridin-2-carbonsäure-5-carbonsäuremethylester

Ausbeute: 60%  Schmelzpunkt: 185 ° C

**Beispiel 5**

Pyridin-2-carbonsäure-4-carbonsäurebutylester

Ausbeute: 41%  Schmelzpunkt: 102 ° C

**Beispiel 6**

Pyridin-2-carbonsäure-5-carbonsäurebutylester

Ausbeute: 51%  Schmelzpunkt: 104 ° C

**Beispiel 7**

Pyridin-2-carbonsäure-4-carbonsäure-(3-methoxybutyl)-ester

Ausbeute: 63%  Schmelzpunkt: 77 ° C

**Beispiel 8**

Pyridin-2-carbonsäure-5-carbonsäure-(3-methoxybutyl)-ester

Ausbeute: 25%  Schmelzpunkt: 163 ° C

## Patentansprüche

**1.** 4- oder 5-substituierte Pyridin-2-carbonsäuren der Formel I

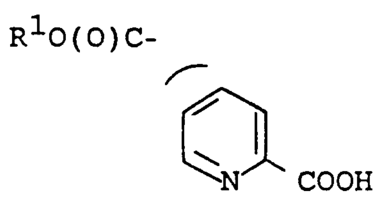

(I)

worin

$R^1$ $C_1$-$C_{12}$-Alkyl, $C_2$-$C_{12}$-Alkenyl, $C_2$-$C_{12}$-Alkynyl, nichtbenzoanneliertes oder benzoanneliertes $C_5$-$C_7$-Cycloalkyl, Aryl oder Heteroaryl bedeutet, wobei dieses für $R^1$ genannten Rest unsubstituiert ist oder substituiert ist mit einem oder mehreren gleichen oder unterschiedlichen Resten $R^2$, wobei

$R^2$ Halogen, Hydroxy, Cyano, Nitro, Nitroxy, Amino, Carboxyl, $C_1$-$C_4$-Alkoxy, $C_1$-$C_4$-Alkoxycarbonyl, $C_1$-$C_4$-Alkyl- oder dialkylamino, Indolyl oder Phenyl bedeutet, wobei der Indolyl- und Phenylrest unsubstituiert ist oder 1-, 2- oder 3-fach substituiert ist mit Halogen, Nitro, $C_1$-$C_4$-Alkyl oder $C_1$-$C_4$-Alkoxy, wobei bei Mehrfachsubstitition die Reste gleich oder verschieden sind sowie die physiologisch verträglichen Salze, wobei die 4-Methyl- und -Benzylester der 2,4- und die 5-Methyl- und -Benzylester der 2,5-Pyridindicarbonsäure ausgenommen sind.

**2.** Verbindungen nach Anspruch 1, worin
$R^1$ $C_1$-$C_6$-Alkyl, $C_2$-$C_6$-Alkenyl, $C_2$-$C_6$-Alkynyl, $C_5$-$C_7$-Cycloalkyl, Aryl oder Heteroaryl bedeutet, wobei diese für $R^1$ genannten Reste unsubstituiert sind oder substituiert sind mit einem oder zwei gleichen oder unterschiedlichen Resten $R^2$, wobei
$R^2$ Halogen, Hydroxy, Cyano, Amino, Carboxyl, $C_1$-$C_4$-Alkoxy, $C_1$-$C_4$-Alkoxycarbonyl, $C_1$-$C_4$-Alkyl- oder dialkylamino, oder Phenyl bedeutet, wobei der Phenylrest unsubstituiert ist oder 1-fach substituiert ist mit Halogen, $C_1$-$C_2$-Alkyl oder $C_1$-$C_2$-Alkoxy, sowie die physiologisch verträglichen Salzen, wobei die 4-Methyl- und -Benzylester der 2,4- und die 5-Methyl- und -Benzylester der 2,5-Pyridindicarbonsäure ausgenommen sind.

**3.** Verbindungen nach Anspruch 1, worin
$R^1$ $C_1$-$C_5$-Alkyl, $C_6$-Cycloalkyl, Phenyl oder Pyridyl bedeutet, wobei diese für $R^1$ genannten Reste unsubstituiert sind oder substituiert sind mit einem oder zwei gleichen Resten $R^2$, wobei
$R^2$ Hydroxy, Amino, Carboxyl, $C_1$-$C_4$-Alkoxy, $C_1$-$C_4$-Alkoxycarbonyl, oder Phenyl bedeutet, wobei der Phenylrest unsubstituiert ist oder 1-fach substituiert ist mit Methyl oder Methoxy, sowie die physiologisch verträglichen Salze, wobei die 4-Methyl- und -Benzylester der 2,4- und die 5-Methyl- und -Benzylester der 2,5-Pyridindicarbonsäure ausgenommen sind.

**4.** Verfahren zur Herstellung von 4- oder 5-substituierten Pyridin-2-carbonsäuren der Formel I sowie deren physiologischen verträglichen Salze zuzüglich der 4-Methyl- und -Benzylester der 2,4- und die 5-Methyl- und -Benzylester der 2,5-Pyridindicarbonsäure, dadurch gekennzeichnet, daß man eine Verbindung der allgemeinen Formel II

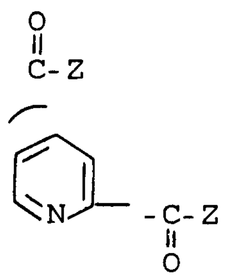

(II),

worin
Z Halogen, insbesondere Chlor oder Brom, Hydroxy oder Alkoxy, insbesondere ($C_1$-$C_6$)-Alkoxy, bevorzugt ($C_1$-$C_4$-)Alkoxy, bedeutet
mit einer Verbindung der allgemeinen Formel III

$HO-R^1$ (III)

umsetzt,
worin
$R^1$ die in Anspruch 1 angegebene Bedeutung hat, und in einer zweiten Reaktionsstufe den daraus enstandenen Diester der allgemeinen Formel IV

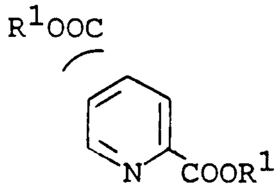

(IV)

in 2-Stellung selektiv verseift.

**5.** Verbindungen nach Anspruch 4 zur Inhibierung der Prolin- und Lysinhydroxylase.

6. Verbindungen nach Anspruch 4 zur Anwendung als Fibrosuppressiva und Immunsuppressiva.

7. Arzneimittel, enthaltend eine Verbindung nach Anspruch 4 und/oder deren physiologisch verträglichen Salze zur Behandlung von Störungen der Biosynthese von Kollagen und kollagenähnlichen Stoffen bzw. der Biosynthese von C1q.

8. Verfahren zur Herstellung von Arzneimitteln zur Beeinflussung der Biosynthese von Kollagen und kollagenähnlichen Stoffen bzw. der Biosynthese von C1q, dadurch gekennzeichnet, daß man in das Arzneimittel eine Verbindung der Formel I nach Anspruch 4 und/oder ein physiologisch verträgliches Salz dieser Verbindung einverleibt.

**Patentansprüche für folgenden Vertragsstaat : ES**

1. Verfahren zur Herstellung von 4- oder 5-substituierten Pyridin-2-carbonsäuren der Formel I

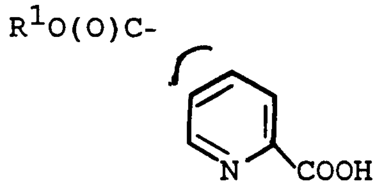

(I)

worin

$R^1$ $C_1$-$C_{12}$-Alkyl, $C_2$-$C_{12}$-Alkenyl, $C_2$-$C_{12}$-Alkynyl, nichtbenzoanneliertes oder benzoanneliertes $C_5$-$C_7$-Cycloalkyl, Aryl oder Heteroaryl bedeutet, wobei dieses für $R^1$ genannten Rest unsubstituiert ist oder substituiert ist mit einem oder mehreren gleichen oder unterschiedlichen Resten $R^2$, wobei

$R^2$ Halogen, Hydroxy, Cyano, Nitro, Nitroxy, Amino, Carboxyl, $C_1$-$C_4$-Alkoxy, $C_1$-$C_4$-Alkoxycarbonyl, $C_1$-$C_4$-Alkyl- oder dialkylamino, Indolyl oder Phenyl bedeutet, wobei der Indolyl- und Phenylrest unsubstituiert ist oder 1-, 2- oder 3-fach substituiert ist mit Halogen, Nitro, $C_1$-$C_4$-Alkyl oder $C_1$-$C_4$-Alkoxy, wobei bei Mehrfachsubstitition die Reste gleich oder verschieden sind sowie die physiologisch verträglichen Salze, wobei die 4-Methyl- und -Benzylester der 2,4- und die 5-Methyl- und -Benzylester der 2,5-Pyridindicarbonsäure ausgenommen sind, dadurch gekennzeichnet, daß man eine Verbindung der allgemeinen Formel II

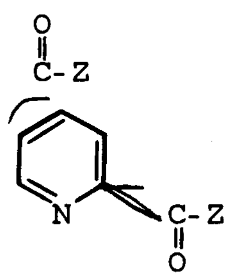

(II),

worin

Z Halogen, insbesondere Chlor oder Brom, Hydroxy oder Alkoxy, insbesondere ($C_1$-$C_6$)-Alkoxy, bevorzugt ($C_1$-$C_4$-)Alkoxy, bedeutet

mit einer Verbindung der allgemeinen Formel III

HO-$R^1$ (III)

umsetzt,

worin

$R^1$ die in Anspruch 1 angegebene Bedeutung hat, und in einer zweiten Reaktionsstufe den

daraus enstandenen Diester der allgemeinen Formel IV

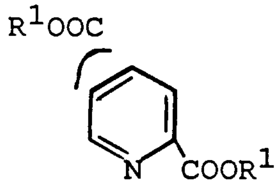

(IV)

in 2-Stellung selektiv verseift.

2. Verfahren nach Anspruch 1, worin
   $R^1$ $C_1$-$C_6$-Alkyl, $C_2$-$C_6$-Alkenyl, $C_2$-$C_6$-Alkynyl, $C_5$-$C_7$-Cycloalkyl, Aryl oder Heteroaryl bedeutet, wobei diese für $R^1$ genannten Reste unsubstituiert sind oder substituiert sind mit einem oder zwei gleichen oder unterschiedlichen Resten $R^2$, wobei
   $R^2$ Halogen, Hydroxy, Cyano, Amino, Carboxyl, $C_1$-$C_4$-Alkoxy, $C_1$-$C_4$-Alkoxycarbonyl, $C_1$-$C_4$-Alkyl- oder dialkylamino, oder Phenyl bedeutet, wobei der Phenylrest unsubstituiert ist oder 1-fach substituiert ist mit Halogen, $C_1$-$C_2$-Alkyl oder $C_1$-$C_2$-Alkoxy, sowie die physiologisch verträglichen Salzen, wobei die 4-Methyl- und -Benzylester der 2,4- und die 5-Methyl- und -Benzylester der 2,5-Pyridindicarbonsäure ausgenommen sind.

3. Verfahren nach Anspruch 1, worin
   $R^1$ $C_1$-$C_5$-Alkyl, $C_6$-Cycloalkyl, Phenyl oder Pyridyl bedeutet, wobei diese für $R^1$ genannten Reste unsubstituiert sind oder substituiert sind mit einem oder zwei gleichen Resten $R^2$, wobei
   $R^2$ Hydroxy, Amino, Carboxyl, $C_1$-$C_4$-Alkoxy, $C_1$-$C_4$-Alkoxycarbonyl, oder Phenyl bedeutet, wobei der Phenylrest unsubstituiert ist oder 1-fach substituiert ist mit Methyl oder Methoxy, sowie die physiologisch verträglichen Salze, wobei die 4-Methyl- und -Benzylester der 2,4- und die 5-Methyl- und -Benzylester der 2,5-Pyridindicarbonsäure ausgenommen sind.

4. Verbindungen nach Anspruch 1 zur Inhibierung der Prolin- und Lysinhydroxylase.

5. Verbindungen nach Anspruch 1 zur Anwendung als Fibrosuppressiva und Immunsuppressiva.

6. Arzneimittel, enthaltend eine Verbindung nach Anspruch 1 und/oder deren physiologisch verträglichen Salze zur Behandlung von Störungen der Biosynthese von Kollagen und kollagenähnlichen Stoffen bzw. der Biosynthese von C1q.

7. Verfahren zur Herstellung von Arzneimitteln zur Beeinflussung der Biosynthese von Kollegen und kollagenähnlichen Stoffen bzw. der Biosynthese von C1q, dadurch gekennzeichnet, daß man in das Arzneimittel eine Verbindung der Formel I nach Anspruch 1 und/oder ein physiologisch verträgliches Salz dieser Verbindung einverleibt.